Europäisches Patentamt

**European Patent Office**

**Office européen des brevets**

(19)

(11) Publication number: **0 247 959**

**A2**

(12)

# EUROPEAN PATENT APPLICATION

(21) Application number: **87500029.1**

(22) Date of filing: **27.05.87**

(51) Int. Cl.⁴: **A 61 B 5/02**

(30) Priority: **27.05.86 ES 555887**

(43) Date of publication of application:
**02.12.87 Bulletin 87/49**

(84) Designated Contracting States:
**AT BE CH DE FR GB GR IT LI LU NL SE**

(71) Applicant: **Valentines de la Iglésia, José Ramon**
**Elisenda de Montcada, 56**
**E-08330 Prémia de Mar Barcelona (ES)**

(72) Inventor: **Valentines de la Iglésia, José Ramon**
**Elisenda de Montcada, 56**
**E-08330 Prémia de Mar Barcelona (ES)**

(74) Representative: **Gomez-Acebo y Pombo, José Miguel**
**c/o CLARKE, MODET & Co. Balmes, 191**
**E-08006 Barcelona (ES)**

(54) **Warning and/or alarm system for detecting the recuperation of vital constants in individuals diagnosed as clinically dead.**

(57) Warning and/or alarm system for detecting the recuperation of vital constants in individuals diagnosed as clinically dead, characterised because it is applied to the supposed cadaver a detector-warning assembly consisting of means for transducing determined vital functions, inside or outside of the body; first means of amplifying the signals received from the transducers; first means of providing audible and/or visual warnings, capable of being activated by the signals coming from the first means of amplification; and first means of providing a supply of energy to the assembly. The system comprises means of distance transmission of the signals received from the transducers; means of reception to receive these remotely produced signals; second means of providing audible and/or visual warnings, which are capable of being activated by signals coming from the said means of reception; and, possibly, second means of providing a supply of energy to the said means of reception and second means of warning. Said first and/or second means of supplying energy are equipped with, preferentially, a blocking device, which must necessarily be removed or deactivated to permit the supply of energy to commence.

FIG. 1

## Description

### "WARNING AND/OR ALARM SYSTEM FOR DETECTING THE RECUPERATION OF VITAL CONSTANTS IN INDIVIDUALS DIAGNOSED AS CLINICALLY DEAD"

This invention concerns a warning and/or alarm system for the detection of any sign of recuperation in the vital functions of persons considered to be clinically dead.

Once a person is considered to be clinically dead and this circumstance is assumed by the relatives and/or professional persons in the charge of whom is the supposedly deceased,it is most natural that it be considered to be useless to continue supervising his state.

Despite the present systems available for verifying clinical death, the applicant, after statistical research and consulting extensive national and international information, has reached the conclusion that two persons in five hundred considered clinically dead, are in a state of apparent death. It is further known that such cases have in fact come to be buried.

Nevertheless, in spite this relatively high proportion of cases of apparent death, there exists no known means by which it has been attempted to alleviate this situation.

This invention comes therefore to fill a considerable void that is observable among the security arrangements established worldwide.

Essentially, the subject system of this invention is characterised because, to the supposed cadaver, is applied a detector-warning assembly formed by means for transducing determined vital functions inside and outside the body; a first means for amplifying the signals received from the transducers; a first means for providing visual and/or audible warning, capable of being activated by the signals coming from the first amplifying means; and a first means of supplying energy to the assembly.

A further characteristic of the invention is the provision of means to transmit the signals received from the transducers over a distance; a second means for visual and/or audible warning capable of being activated by signals coming from the said means of reception; and, possibly, a second means for supplying energy to the said means of reception and said means of warning.

In accordance with another characteristic of the invention, the said first and/or second means of supplying energy are preferentially provided with a blocking device which must necessarily be removed or disactivated to permit the start of supplying energy.

The duration of the supply system shall be predetermined.

Other characteristics and advantages of the system for warning and/or alarm subject of this invention, shall become evident from the description given below in conjunction with the drawings attached, which illustrate, by way of a non-limiting example, one way of establishing the said system.

Figures 1 and 2 provide two examples of estabhishing the detector-warning assembly and a second warning element;

Figure 3 shows, in perspective, a schematic view of a mortuary with the warning and/alarm system incorporated; and

Figure 4 is a highly schematic plan view of how the system could be applied to a cementery.

In these drawings it can be appreciated that the warning and/or alarm system described comprises a detector-warning assembly 16, prepared for application to the suposed cadaver and formed by means 1 for the transducing of determined vital functions, first means of amplification 2 for the signals received from the transducers 1, first means of audible warning devices 3 and first means of visual warning devices 4, capable of being activated by the first means of amplification 2, and first means of supplying energy 5, for the assembly.

The said means of transducing 1 may preferentially be constituted by electrodes similar to those for making electroencephalograms and electrocardiograms, as well as also being able to be constituted by probes of any type.

The first detector-warning assembly 16 is conceived to be preferentially applied to the body of the supposed cadaver, in a clearly visible spot, in such a way that it consitutes a type of alarm or warning able to be observed within a zone relatively close to the supposed cadaver. Clearly this means it can only be used by itself in those cases where there is always some one near to the supposedly deceased and, of course, only before the burial of the cadaver.

In the cases where the supposedly deceased may have to remain alone for some time or at some distance from the supervision of some one, it is not sufficient to use only the detector-warning assembly 16. In such a case, use is made of a second warning device 19, in conjunction with means 6 of transmitting the signals received from the transducers 1 over a distance. The said device 19 comprises means of reception 7 to receive the signals emitted by the means 6, second means of audible warning 8, second means of visual warning 9 and and, possibly, second means of suypplying energy 10 to the said means of reception 7 and the second means of warning 8 and 9.

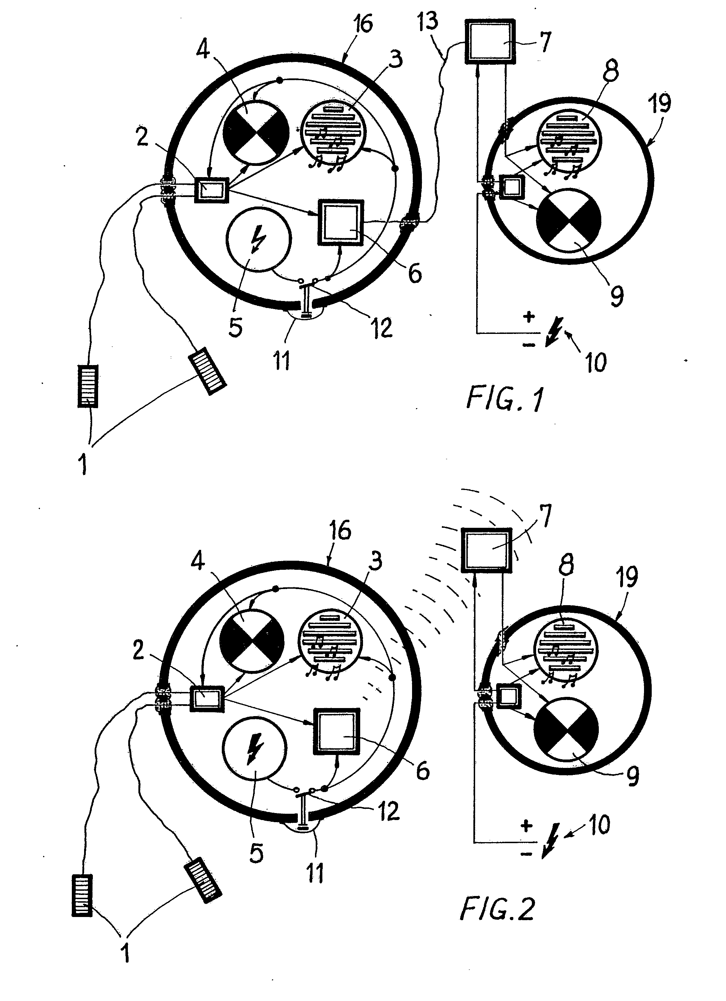

The said first and/or second means of supplying energy 5 and 10, are equipped preferentially with a blocking device that has necessarily to be removed and deactivated to allow the supply of energy to commence. In particular, in Figures 1 and 2 of the drawings it can be appreciated that the said blocking device of the first means of supplying energy 5 incorporates a seal 11 that has to be withdrawn in order to activate the switch 12 which permits the means of supplying energy to be made active.

In Figure 1, the said means of transmitting to a distance admit the use of signal transmitting cables 13, while Figure 2 contemplates the means 16 for transmitting the said signals wirelessly to the receiver 7.

Figure 5 shows mortuary consisting of a number of refrigerated deposits 14 and a group of tables 15 where the supposed deceased are kept. To each of the deceased is applied a detector-warning assembly 16, which, by means of cables or by wireless transmission, activates the control panel 18 and the warning device 191.

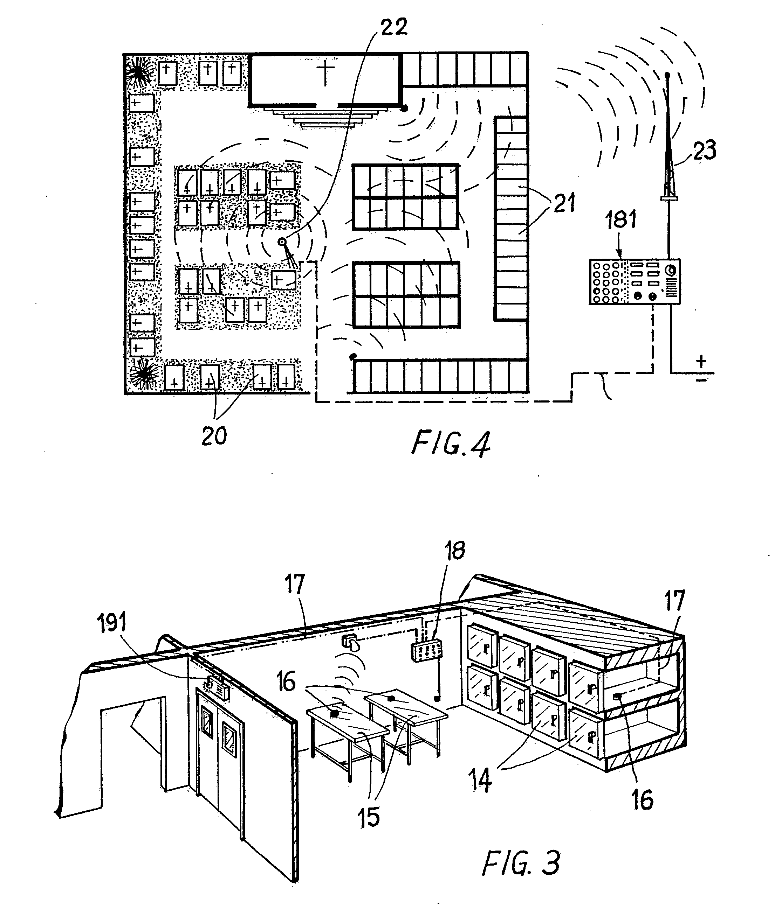

Figure 4 shows, in outline, how the subject system can be applied in the case of a cemetery. The supposed cadavers contained in tombs 20 and in niches 21, each equipped with a detector-warning assembly 16, are monitored from a data receiving post 181 at which arrive the signals sent from each of the assemblies 16 via cables 171 or else via a wireless path through the repeater 22, for long distances, and the antenna 23 of means of reception 7 contained in panel 181. Clearly each of the signals from assemblies 16 must be individually identificable in order to rapidly locate the site occupied by the supposed cadaver to which pertains the assembly 16 which has emitted some signal.

Having described sufficiently the nature of the device, as well as the manner of making use of it, it is hereby placed on record that all which does not alter, change or modify its basic principle, may be subject to variations in detail.

**Claims**

1- Warning and/or alarm system for detecting the recuperation of vital constants in individuals diagnosed as clinically dead, characterised because it is applied to the supposed cadaver a detector-warning assembly consisting of means for transducing determined vital functions, inside or outside of the body; first means of amplifying the signals received from the transducers; first means of providing audible and/or visual warnings, capable of being activated by the signals coming from the first means of amplification; and first means of providing a supply of energy to the assembly.

2.- Warning and/or system according to claim 1, characterised because it has means of distance transmission of the signals received from the transducers; means of reception to receive these remotely produced signals; second means of providing audible and/or visual warnings, which are capable of being activated by signals coming from the said means of reception; and, possibly, second means of providing a supply of energy to the said means of reception and second means of warning.

3.- Warning and/or alarm system according to claims 1 and 2, characterised because the said first and/or second means of supplying energy are equipped with, preferentially, a blocking device, which must necessarily be removed or deactivated to permit the supply of energy to commence.

4.- Warning and/or alarm system according to claims 1 and 2, characterised because the mentioned means of distance transmission are wireless transmission means.

5.- Warning and/or alarm according to claims 1 and 2, characterised because the mentioned means of distance transmission are wire transmission means.

FIG. 1

FIG.2

0247959

FIG.4

FIG. 3